# EUROPEAN PATENT APPLICATION

(11) **EP 0 947 582 A1**
(43) Date of publication of application: **06.10.1999**
(21) Application number: 98870065.4
(22) Date of filing: 31.03.1998
(51) Int. Cl.: C12N 15/12, C07K 14/705, C07K 19/00, G01N 33/68, C12N 15/10

(54) **A polypeptide structure for use as a scaffold**

(71) Applicant: INNOGENETICS N.V., 9052 Gent (BE)
(72) Inventor: Desmet, Johan, 8500 Kortrijk (BE); Hufton, Simon, 6228 BJ Maastricht (NL); Hoogenboom, Hendricus, 6214 AE Maastricht (NL); Sablon, Erwin, 1785 Merchtem (BE)

(57) **Abstract**

The present invention relates to a polypeptide chain having a β sandwich architecture which can be used as a scaffold, i.e. a supporting framework, carrying antigen- or receptor-binding fragments. More specifically, the present invention describes a non-glycosylated CTLA4-like β sandwich carrying B7-binding fragments.

## Description

### FIELD OF THE INVENTION

The present invention relates to a polypeptide chain having a β sandwich architecture which can be used as a scaffold, i.e. a supporting framework, carrying antigen- or receptor-binding fragments. More specifically, the present invention relates to a non-glycosylated CTLA4-like β sandwich carrying B7-binding fragments.

### BACKGROUND OF THE INVENTION

It is clear from the literature that the primary amino acid sequence of a protein determines its three-dimensional (3D) structure, which in turn determines protein function. In most cases the 3D structure is essentially uneffected by amino acids at loci where the amino acid side chain is directed towards the solvent. Loci where limited variety is allowed have the side group directed toward other parts of the protein. This general rule, however, does not hold for those proteins whose function consists of, or relies upon binding with other biomolecules. Binding of such proteins to other molecules is non-covalently but often very tightly and specifically. In such instance, the identity of residues of the interacting domains that point towards the solvent becomes very important as well as the 3D-structure that they adopt, because binding results from complementarity of the surfaces that come into contact: bumps fit into holes, dipoles align and hydrophobic residues contact other hydrophobic residues. Individual water molecules eventually can fill a well determined space in intermolecular interfaces, and then usually form hydrogen bonds to one or more atoms of the protein or other biomolecules or to other bound water.

The 3D structure can often be predicted on the basis of the primary amino acid sequence. A major obstacle towards the correct prediction of the entire protein structure is the modelling of "loops". These are regions of the polypeptide chain that have a lower degree of structural and sequential regularity and where insertions and deletions often occur during evolution. Yet, this diversity does not fundamentally alter the core structure of the remaining polypeptide chain. Often such loops, which are on the outside of the protein structure, will determine the interaction with other biomolecules. The conformation of loop fragments is largely controlled by the attachment of their ends to fragments that constitute the framework of the molecule, for instance secondary structural units. This is also the basic idea when grafting such polypeptide chains onto the core of the protein structure which functions as a scaffold. Scaffolds, which are able to restrain the 3D structure of a polypeptide chain within a loop, can thus be of utmost importance.

Immunoglobulins are multi domain proteins consisting of a heavy and a light chain. The variable domain of immunoglobulins is composed of a antigen binding site formed by six loops clustered in space and carried by two domains that adopt a so called-β sandwich structure (one β domain from the heavy chain carrying 3 loops and one from the light chain carrying 3 loops) (Amit et al, 1986). The high sequence variability of these loops allows immunoglobulins to recognise a variety of antigens. Though this sequence variation does determine in part the structure of the loops and the antigen specificity, the gross structure of the immunoglobulin variable domain's framework is hereby not altered. Reversely, the latter β-domains, consisting of anti-parallel strands that are connected by the loops, function as a scaffold that strongly determines the three dimensional structure of the loops by constraining their structure and providing a carrier function (Amzel,L.M. and Poljak,RJ., 1979; Williams A.F.,1987). Furthermore, the β-sandwich architecture of both the heavy and light chains have a roughly half-cylindrical structure, with a more or less open side. In functional immunoglobulins both domains (heavy and light chain) are packed onto eachother thereby forming a barrel-like fold.

A serious drawback in using the immunoglobulin scaffold to restrain random polypeptide sequences, however, is that the core β-sandwich is composed of two chains (the heavy and light chains) which interact, thereby bringing the variable loops of both chains (6 in total) in close proximity. This complicates the use of immunoglobulins for restraining the conformation of randomized polypeptide sequences such as in antibody libraries on filamentous phage. Large peptide libraries on phage are described in US 5223409 to Ladner et al. and US 5571698 to Ladner et al. In the latter system, each randomized peptide is fused to the gene III protein of the M13 phage. In this regard, it is clear that a large, multi-domain protein is not ideal in phage-derived selection of peptidic leads. Consequently, efforts have been devoted to design novel, smaller and simpler proteins, based on the structure of the variable domain of an antibody. One of these efforts is the construction of a protein, named minibody, which consists of a fusion of the variable regions of an antibody and the CH3 domain of a immunoglobulin, all in a single polypeptide. Minibodies spontaneously dimerize to form an antibody fragment that is bivalent. The minibody scaffold, which contains a number of residue modifications with respect to the original immunoglobulin sequence, can be obtained both by solid-phase synthesis and by expression in bacteria (Bianchi et al, 1993; Bianchi et al, 1994). Further modifications of the latter molecule were Successful in improving some important properties, for example its solubility in aqueous media (Bianchi et al ,1994). Other attempts to simplify the existing multi domain immunoglobulin molecule have resulted in recombinant fragments, made of two forms: Fv fragments and Fab's. Fv fragments are heterodimers composed of a variable heavy chain (V_{H}) and a variable light chain (V_{L}) domain and are the smallest functional fragments of antibodies that maintain the binding and specificity of the whole antibody. In these constructs, the immunoglobulin domains forming the characteristic β-barrel can still interact with each other. However, Fv fragments are unstable. Stable Fv's have been produced by making recombinant molecules in which the V_{H} and V_{L} domains are connected by a peptide linker so that the antigen-binding site is regenerated in a single molecule. These recombinant molecules are termed single chain Fv's (scFv's; Raag and Whitlow, 1995). The V_{H}-V_{L} heterodimer can alternatively be stabilized by an interchain disulfide bond and is then termed dsFvs (Reiter et al, 1994). Fab fragments, on the other hand, are composed of the light chain and the heavy chain Fd fragment (V_{H} and CH1) connected to each other via the interchain disulfide bond between CL and CH1.

Taken together, each simplification of the otherwise complex multidomain immunoglobulin molecule still results in a unit wherein two immunoglobulin folds interact with each other thereby reconstituting the antigen binding site which consists of two pairs of three loops each grafted onto a separate β-sandwich framework.

Another drawback for using immunoglobulins to restrain randomized polypeptide sequences is the fact that the antigen-binding site of the latter molecules, which is constituted of six clustering loops, may not be able to bind small and/or cryptic sites within molecules. In other words, steric hindrance during the screening or selection for proteins that bind small and/or cryptic sites may occur when using an immunoglobulin framework. Solutions to the latter problem have been offered by restraining randomized polypeptide sequences of variable length by means of cyclization upon for instance incorporation of cysteine residues at fixed positions forming a disulfide bond, or, by means of incorporating a metal binding motif or a conserved hydrophobic core into the polypeptide, or, by means of embedding peptide segments in "small proteins" with a defined 3D structure such as endothelin, trefoil proteins, guanylin and the like (Ladner, 1995; Cannon et al. 1996). While these solutions have been very elegant, they result in small peptides which may rather quickly be cleared from the bloodstream, thus posing problems for certain therapeutic purposes.

The present invention relates to an alternative, simpler version of a monomeric polypeptide, compared to antibody-derived and small protein scaffolds, which can, to our surprise, efficiently be used as a scaffold.

### AIMS OF THE INVENTION

The more tightly a polypeptide segment is constrained, the more likely this segment will bind to its target with high specificity and affinity (Ladner, 1995). In other words, there is a need to design scaffolds which are able to carry, or are embedded with, polypeptides efficiently binding to an antigen or receptor. Already existing scaffolds, such as antibody-derived scaffolds and "small proteins", are still faced with particular limitations. Indeed, antibody-derived scaffolds containing 6 loops constituting the antigen-binding site, are composed of rather large and complex dimers which, due to steric hindrance, may not be usefull for carrying polypeptides binding small and/or cryptic sites. Furthermore, large molecules containing an antigen binding site composed of six loops have a relatively higher chance to bind in a non-specific manner, or to cross-react, compared to smaller and simpler molecules containing an antigen binding site composed of less than six loops (Maclennan, 1995). Moreover, antibody-derived scaffolds have the tendency to denature more easily during the aggressive purification protocols required for biotherapeutic production (Maclennan, 1995). "Small proteins", on the other hand, may be quickly cleared from the bloodstream which is not desirable for certain therapeutic purposes. Furthermore, both antibody-derived -and "small protein" scaffolds, which are used to generate large peptide libraries using phage-display technology, are limited by the fact that phage display is not able to display active forms of proteins that require eukaryotic-specific posttranslational modifications (such as glycosylation) for activity (Cannon et al, 1996). Also Metzler et al (1997) indicated that glycosylation is important for binding activity, structural integrity and solubility of CTLA-4.

In order to overcome the above-indicated limitations, it is an aim of the present invention to provide an alternative, simplier and preferably unglycosylated scaffold which is not easily cleared from the blood stream.

It is also an aim of the present invention to provide a scaffold structure which is sufficiently stable to allow grafting of polypeptide chains as loops onto said scaffold without substantial alteration of the scaffold structure.

It is further an aim of the present invention to provide a scaffold structure that can restrain randomized polypeptide sequences thereby determining the 3D-structure of said polypeptide sequences.

It is also an aim of the present invention to provide a scaffold structure wherein at least one, preferably two and more preferably three of the restrained polypeptide loops are constituting a binding domain for other molecules.

It is also an aim of the present invention to provide a scaffold structure wherein at least two, preferably three, more preferably four, more preferably five and even more preferably six of the restrained polypeptide loops are constituting at least two binding domains for other molecules.

It is also an in of the present invention to provide a scaffold structure which is soluble under physiological conditions.

It is also an aim of the present invention to provide an unglycosylated scaffold structure that remains soluble and/or functional in a bacterial background. In this regard, it should be clear, in view of the prior art (i.e. see Cannon et al., 1996, and, Meltzer et al, 1997) which indicates that unglycosylated molecules may aggregate and/or may have a reduced bioactivity, that a person skilled in the art is guided away to use an unglycosylated scaffold.

It is also an aim of the present invention to provide a scaffold structure that is essentially functional as a monomeric protein.

All these aims are met by the following embodiments of the present invention.

### BRIEF DESCRIPTION OF DRAWINGS

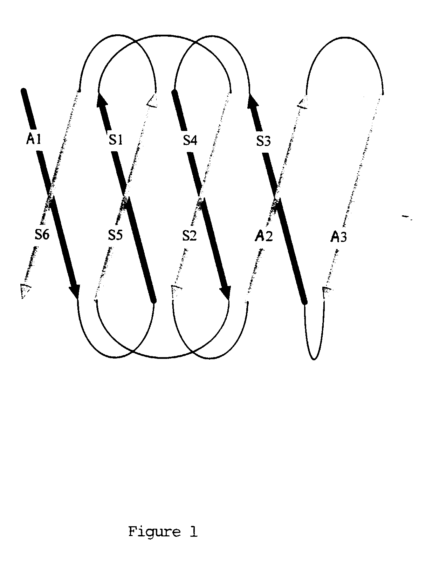

**Figure 1** shows a schematic representation of the scaffold of the present invention. The six basic β-strands are named S1, S2, S3, S4, S5 and S6; the three additional or optional β-strands are named A1, A2 and A3. The loops connecting the strands are indicated by a thin, black, curving line.

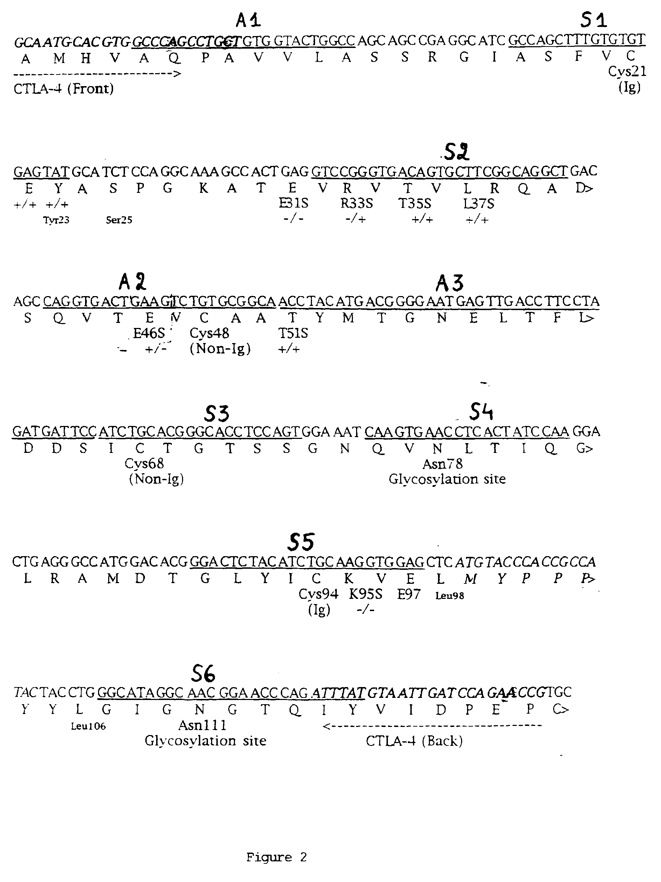

**Figure 2** shows the sequence coding for SCA, which is based on the extracellular part of human CTLA-4 gene as described by Metzler *et al.* (1997) but without the C-terminal cysteine residue (Cys 123). β-sheets are underlined, Cysteine residues and glycosylation sites are shown. Mutations and there effect on CD80/86 binding are also indicated.
**Figure 3** indicates the specific primers used to clone SCA as a *Apa*L1/*Not*1 fragment.
**Figure 4** shows a shematic representation of the Phagemid pCES1 construct. Antibody genes: V_{L}-C_{L}, variable (V) and constant (C) region of the light chain; V_{H}-C_{H1}, variable and first constant region of the heavy chain; *PlacZ,* promoter; rbs, ribosome binding site; S, signal sequence; H6, six histidines stretch for IMAC purification; tag, *c-myc-*derived tag; amber, amber codon that allows production of soluble Fab fragments in non-suppressor strains, gIII, gene encoding one of the minor coat proteins of filamentous phage. Restriction sites used for cloning are indicated.
**Figure 5** demonstrates the expected patterns of the *Bst*N1 fingerprints of clones with the correct sized insert.
**Figure 6** demonstrates a strong signal showing binding to both B7.1-Ig and B7.2-Ig and not to BSA or plastic. The latter signal also decreases proportionately with the amount of phage present.
**Figure 7** shows an SDS-page analysis with DTT (lanes 1 and 3) or without DTT (lanes 2 ad 4) followed by detection with anti-CTLA-4 (lanes 1 and 2) or with anti-myc (lanes 3 ad 4). which demonstrates that part of the expressed 16 kD SCA protein is present in the medium. The 83 kD band is probably due to a fusion of the SCA protein with the gene 3 coat protein of the phage.

### DETAILED DESCRIPTION OF THE INVENTION

The invention described herein draws on previously published work and pending patent applications. By way of example, such work consists of scientific papers, patents or pending patent applications. All these publications and applications, cited previously or below are hereby incorporated by reference.

The present invention relates to a scaffold composed of a single-chain polypeptide having the following structural properties:
- it contains at least two cysteine residues which form at least one disulphide bond, and
- it possesses less than 10% of alpha helical conformation, and
- it contains at least six β-strands (S1, S2, S3, S4, S5, S6) which:
   - are connected by amino acid loops of variable conformation and lenght according to the following topology: S1-S2-S3-S4-S5-S6, and
   - form two β-sheets (one formed by S1/S4/S3 and one formed by S6/S5/S2 wherein the symbol "/" denotes the hydrogen bonding interactions between two spatially adjacent β strands) which are each characterized by an anti-parallel arrangement of said β strands and which are packed onto eachother so that they form a β sandwich architecture. Furthermore, the present invention regards a scaffold as defined above
   which may contain a maximum of three additional β strands (A1, A2 and A3), and, which has the following topology: A1-S1-S2-A2-A3-S3-S4-S5-S6, and, which possesses the following β-sheets: A1/S1/S4/S3 and S6/S5/S2/A2/A3.

The present invention also relates to a scaffold as defined above wherein:
- A1 is the amino acid sequence AQPAVVLA (SEQ ID 1) or any functionally equivalent derivative of said sequence,
- S1 is the amino acid sequence ASFPVEY (SEQ ID 2) or any functionally equivalent derivative of said sequence,
- S2 is the amino acid sequence EVRVTVLRQA (SEQ ID 3) or any functionally equivalent derivative of said sequence,
- A2 is the amino acid sequence QVTEVCAA (SEQ ID 4) or any functionally equivalent derivative of said sequence,
- A3 is the amino acid sequence TYMMGNELTFLDDS (SED ID 5) or any functionally equivalent derivative of said sequence,
- S3 is the amino acid sequence ICTGTSS (SEQ ID 6) or any functionally equivalent derivative of said sequence,
- S4 is the amino acid sequence QVNLTIQ (SEQ ID 7) or any functionally equivalent derivative of said sequence,
- S5 is the amino acid sequence GLYICKVE ( SEQ ID 8) or any functionally equivalent derivative of said sequence,
- S6 is the amino acid sequence GIGNGTQIY (SEQ ID 9) or any functionally equivalent derivative of said sequence.

It should be clear that the scaffold of the present invention can be prepared by any method known in the art such as classical chemical synthesis, as described by Houbenweyl (1974) and Atherton & Shepard (1989), or by means of recombinant DNA techniques as described by Maniatis et al. (1982).

The term "alpha helical conformation" refers to the conformation of a polypeptide segment where the main chain adopts a fragment of a right-handed helix (phi- and psi-angles both near 60°), wherein all of the C=O and N-H groups of the peptide linkages lay roughly parallel to the axis of the helix, each carbonyl group being hydrogen bonded to the fourth N-H group on up the chain. The number of amino acid per turn of the helix is 3.61. The pitch (repeat distance) of the helix is 0.541 nm.

The term "beta (β) strand" refers to the conformation of a polypeptide segment where the main chain adopts an extended conformation (Phi- and psi-angles around -120° and +120°, respectively) leading to a relatively linear structure. A beta strand can be interrupted by one or two non-extended residues (for instance a beta bulge), while still be considered as a single strand, as long as the principal axes of the two consecutive sub-fragments do not form an angle greater than 90°.

The term "beta (β) sandwich architecture" refers to two beta-sheets that pack against eachother and in which the strands of both sheets form a so-called relative 'twist angle' of around 30° (within the rage 20-50°).

The terms "any functionally equivalent derivative of said sequences" refer to any variant or fragment of the amino acid (aa) sequences represented by SEQ ID 1 to 9 which does not result in a significant alteration of the global structural and functional properties of the scaffold of the present invention. The latter terms include, post-translational modifications of the aa sequences represented by SEQ ID 1 to 9 such as glycosylation, acetylation, Phosphorylation, modifications with fatty acids and the like. Included within the definition are, for example, amino acid sequences containing one or more analogues of an aa (including unnatural aa's), amino acid sequences with substituted linkages, mutated versions of the amino acid sequences, peptides containing disulfide bonds between cysteine residues, biotinylated amino acid sequences as well as other modifications known in the art. Included within the definition are also those single amino acid substitutions which can further improve the solubility of the unglycosylated protein. Examples of equivalent derivatives, which are not intended to limit the scope of the present invention but are purely illustrative, are:
- for A1, the amino acid sequences TQPSVVLA (SEQ ID 10), TQPPVVLA (SEQ ID 11), SQPAVVLA (SEQ ID 12), KQSPLLVVD (SEQ ID 13), KQSPMLVVN (SEQ ID 14), KQSPMLVAY (SEQ ID 15), KQLPRLVVY (SEQ ID 16), AQRPLLIVA (SEQ ID 17), TQSPAIMSA (SEQ ID 18), QESGPGLV (SEQ ID 19) and the like,
- for S1, the amino acid sequences ASFPCEY (SEQ ID 20), ASFSCEY (SEQ ID 21), VSLSCRY (SEQ ID 22), VNLSCKY (SEQ ID 23), ATLVCNY (SEQ ID 24, VTMTCSA (SEQ ID 25), VTMTCKS (SEQ ID 26), VTITCKA (SEQ ID 27), VTMSCKS (SEQ ID 28), MKLSCVA (SEQ ID 29), LRLSCAT (SEQ ID 30), LKLSCAA (SEQ ID 31), RKLSCAA (SEQ ID 32), LSITCTV (SEQ ID 33), VKLSCTA (SEQ ID 34), VKISCKA (SEQ ID 35), LSITCTV (SEQ ID 36), VKISCKA (SEQ ID 37), VQISCKA (SEQ ID 38) and the like,
- for S2, the amino acid sequences EVRVTVLRQT (SEQ ID 39), EVRVTVLREA (SEQ ID 40), EFRASLYKGV (SEQ ID 41), EFRASLYKGA (SEQ ID 42), EFRASLHKGL (SEQ ID 43), EFRASLHKGT (SEQ ID 44), EVRMYMYQQK (SEQ ID 45), KNYLTWYQQK (SEQ ID 46), EVRVVWYQQK (SEQ ID 47), NFRLAWYQQK (SEQ ID 48), KNFLAWYQQK (SEQ ID 49), EVRMSWVRQS (SEQ ID 50), EVRMEWVRQP (SEQ ID 51), EVRMSWVRHT (SEQ ID 52), EVRMHWVRQA (SEQ ID 53), EVRVNWVRQP (SEQ ID 54), EVRMNWVKQR (SEQ ID 55), EVRIEWVKQR (SEQ ID 56), EVRVHWVRQS (SEQ ID 57), EVRIEWVKER (SEQ ID 58) and the like,
- for A2, the amino acid sequences QVTEVCAT (SEQ ID 59), QMTEVCAT (SEQ ID 60), QVTEVCAG (SEQ ID 61), QMTEVCAM (SEQ ID 62), SDVEVCVG (SEQ ID 63), SAVEVCVV (SEQ ID 64), SAVEVCAV (SEQ ID 65), SAVEVCFI (SEQ ID 66), SPRLLIYD (SEQ ID 67), SPKLLIYW (SEQ ID 68), PPKLLIYG (SEQ ID 69), GLEWVAEI (SEQ ID 70), RLEWIAAS (SEQ ID 71), RLEWVATI (SEQ ID 72), GLEWVAYI (SEQ ID 73), GLEWLGMI (SEQ ID 74), GLEWIGRI (SEQ ID 75), GLEWIGWI (SEQ ID 76), GLEWLGMI (SEQ ID 77), GLEWIGEI (SEQ ID 78), GLEWIGEI (SEQ ID 79) and the like,
- for A3, the amino acid sequences TFTVKNTLGFLDDP (SEQ ID 80), TFTEKNTVGFLDYP (SEQ ID 81), TYMVEDELTFLDDS (SEQ ID 82), TYTVENELTFIDDS (SEQ ID 83), NGNFTYQPQFRSNAEF (SEQ ID 84), NGNFTYQPQFRPNVGF (SEQ ID 85), NGNFSHPHQFHSTTGF (SEQ ID 86), NGNHSHPLQSHTNKEF (SEQ ID 87), YGNYSQQLQVYSKTGF (SEQ ID 88), NGNYSHQPQFYSSTGF (SEQ ID 89), SWNMTHKINSNSNKEF (SEQ ID 90), TSNLASGVPV (SEQ ID 91), ASTRESGVPD (SEQ ID 92), ASTRHIGVPD (SEQ ID 93), RLNSDNFATHYAESVKG (SEQ ID 94), RNKGNKYTTEYSASVKG (SEQ ID 95), SNGGGYTYYQDSVKG (SEQ ID 96), SSGSSTLHYADTVKG (SEQ ID 97), WGDGNTDYNSALKS (SEQ ID 98), DPANGNIQYDPKFRG (SEQ ID 99), YPGSGNTKYNEKFKG (SEQ ID 100), LPGSGSTNYNEKFKG (SEQ ID 101), WGGGSIEYNPALKS (SEQ ID 102), LPGSGRTNYREKFKG (SEQ ID 103) and the like,
- for S3, the amino acid sequences FCSGTFN (SEQ ID 104), TCIGTSR (SEQ ID 105), TCTGISH (SEQ ID 106), NCDGDFD (SEQ ID 107), NCDGNFD (SEQ ID 108), NCDGKLG (SEQ ID 109), NCTVKVG (SEQ ID 110), NCDGKLG (SEQ ID 111), DCDGKLG (SEQ ID 112), NCRGIHD (SEQ ID 113), RFSGSGS (SEQ ID 114), RFTGSGS (SEQ ID 115), RFAGSGS (SEQ ID 116), KFIISRD (SEQ ID 117), RFIVSRD (SEQ ID 118), RFTISRD (SEQ ID 119), RFTISRD (SEQ ID 120), RLSISFD (SEQ ID 121), KATITAD (SEQ ID 122), KATLTVD (SEQ ID 123), RLSISKD (SEQ ID 124), KATFTAD (SEQ ID 125), KATFTAD (SEQ ID 126) and the like,
- for S4, the amino acid sequences RVNLTIQ (SEQ ID 127), KVNLTIQ (SEQ ID 128), TVTFRLW (SEQ ID 129), TVTFYLK (SEQ ID 130), TVTFYLQ (SEQ ID 131), SVTFYLQ (SEQ ID 132), TVTFYLR (SEQ ID 133), KVIFNLW (SEQ ID 134), SYSLTIS (SEQ ID 135), DFTLSIS (SEQ ID 136), DYTLTIS (SEQ ID 137), DFTLTIS (SEQ ID 138), RLYLQMN (SEQ ID 139), ILYLQMN (SEQ ID 140), TLFLEMT (SEQ ID 141), TLFLQMT (SEQ ID 142), QVFLKMN (SEQ ID 143), TAYLQL (SEQ ID 144), TAYMQLS (SEQ ID 145), QVFLKMN (SEQ ID 146), TATMQLS (SEQ ID 147), QIFLKMN (SEQ ID 148) and the like,
- for S5, the amino acid sequences GLYFCKVE (SEQ ID 149), GLYLCKVE (SEQ ID 150); GLYVCKVE (SEQ ID 151), DIYFCKIE (SEQ ID 151), DIYFCLKE (SEQ ID 152), ATYYCQQW (SEQ ID 153), AVYYCQNN (SEQ ID 154), ALYYCQQH (SEQ ID 155), AVYVCQND (SEQ ID 156), GIYYCVLR (SEQ ID 157), AIYYCARN (SEQ ID 158), GLYYCARR (SEQ ID 159), GMYYCARW (SEQ ID 160), ARYYCARE (SEQ ID 161), AVYYCATK (SEQ ID 162), AVYFCARG (SEQ ID 163), AVYYCARH (SEQ ID 164), AXYYCVSY (SEQ ID 165), AVYVCTRG (SEQ ID 166) and the like,
- for S6, the amino acid sequence GMGNGTQIY (SEQ ID 167), ERSNGTIIH (SEQ ID 168), EKSNGTIIH (SEQ ID 169), EKSNGTVIH (SEQ ID 170), TFGVGTKLE (SEQ ID 171), TFGAGTKLE (SEQ ID 172), TFGGGTKLE (SEQ ID 173), YWGQGTSVT (SEQ ID 174), VWGAGTTVT (SEQ ID 175), YWGRGTLVT (SEQ ID 176), YWGRGTLVT (SEQ ID 177), YWGQGTTLT (SEQ ID 178), YWGQGTTLT (SEQ ID 179), YWGQGTLVT (SEQ ID 180) and the like.

It should be clear that the scaffold of the present invention contains at least six β-strands (S1 to S6) and may, but does not have to, comprise one (A1 or A2 or A3), preferably two (A1 and A2, or, A1 ad A3, or A2 and A3), and most preferably three (A1 and A2 and A3) additional β-strands (A1 to A3).

The terms "amino acid loops of variable conformation and length" refer to any aa Sequence of any lenght and any conformation which is able to connect the β-strands of the current invention. The latter as loops preferably contain at least one aa fragment which binds to a receptor or antigen (see further). In this regard, it should be clear that not all loops need to contain (a) receptor/antigen binding fragment (s). Indeed, one out of the 6 loops my contain an above-indicated fragment; preferably, two out of the 6 loops contain an above-indicated fragment and more preferably, three out of the 6 loops contain an above-indicated fragment. However, also four, five and six out of the 6 loops may contain an above-indicated fragment. In the latter regard, the scaffold of the present invention may contain sets of fragments which are able to bind to more than one, preferably two, different receptors/antigens. For exmaple, the loops which connect S5 and S6, S1 and S2, S3 and S4, and, A2 and A3 contain fragments binding to a tumor antigen or B7.1/B7.2 expressing cells and the loops connecting A3 and S3, S2 and A2, S4 and S5, and, A1 and B contain fragments binding to a toxin able to kill the tumor cell expressing said tumor antigen or said B7.1/B7.2 expressing cells in a manner similar as the one described in WO91/07437 to Pfeundschuh and WO96/40260 to De Boer & De Gast, respectively. Other specific examples of such amino acid loops, which are not intended to limit the scope of the present invention but are purely illustrative, are:
- loops connecting said β-strands A1 and S1: SSHGV (SEQ ID 181), SSRGI (SEQ ID 182), SSRGV (SEQ ID 183), SSRGV (SEQ ID 184), SNE (SEQ ID 185), NNE (SEQ ID 186), DNE (SEQ ID 187), DNA (SEQ ID 188), SPGEK (SEQ ID 189), QPGGS (SEQ ID 190) and the like,
- loops connecting said β-strands S1 and S2: SPSHNTD (SEQ ID 191), ASSHNTD (SEQ ID 192), ASPGKAT (SEQ ID 193), ESSGKAD (SEQ ID 194), ASHGKAT (SEQ ID 195), SYNLLAK (SEQ ID 196), TYNLFSK (SEQ ID 197), SYNLFSR (SEQ ID 198), TYNGTGK (SEQ ID 199), and the like,
- loops connecting said β-strands S2 and A2: ND (SEQ ID 200), DS (SEQ ID 201), GS (SEQ ID 202), NS (SEQ ID 203), PGS (SEQ ID 204), PGQ (SEQ ID 205), PEK (SEQ ID 206), PGK (SEQ ID 207), PDK (SEQ ID 208), PEK (SEQ ID 209), and the like,
- loops connecting said β-strands S3 and S4: ES (SEQ ID 210), GN (SEQ ID 211), NE (SEQ ID 212), KD (SEQ ID 213), GT (SEQ ID 214), DSKS (SEQ ID 215), TSQS (SEQ ID 216), NAKN (SEQ ID 217), NPKN (SEQ ID 218), and the like,
- loops connecting said β-strands S4 and S5: GLRAVDT (SEQ ID 219), GLRAADT (SEQ ID 220), GLRAMDT (SEQ ID 221), GLSAMDT (SEQ ID 222), NLHVNHT (SEQ ID 223), NLDVNHT (SEQ ID 224), NLYVNQT (SEQ ID 225), DLYVNQT (SEQ ID 226), NLFVNQT (SEQ ID 227), NMSASQT (SEQ ID 228), and the like,
- loops connecting said β-strands S5 and S6: LMYPPPYFV (SEQ ID 229), LMYPPPYYL (SEQ ID 230), LMYPPPYYV (SEQ ID 231), FMYPPPYLDN (SEQ ID 232), VMYPPPYLDN (SEQ ID 233), VLYPPPYIDN (SEQ ID 234), VMYPPPYIGN (SEQ ID 235), AMYPPPYVYN (SEQ ID 236), and the like.

The present invention further relates to a scaffold as defined above wherein said amino acid loops comprise fragments binding to a receptor or antigen.

The terms "fragments binding to a receptor or antigen" refer to any possible aa sequence which is part of the said loops (i.e. said fragments comprise maximally as many aa's as the loop itself and comprise, more frequently, less aa's than the loop wherefrom they are derived) and which binds any receptor or antigen. In this regard, it should be clear that the scaffold of the present invention can carry any randomized aa sequence. More specifically, the present invention concerns a scaffold as defined above wherein said amino acid loops comprise fragments binding B7.1 and/or B7.2 (see further in the *Examples* section). A detailed description of both B7 molecules and their ligands is given in EP 97870092.0 to Lorré et al.

The present invention concerns a single-chain polypeptide as defined above for use as a scaffold In other words, the scaffold of the present invention can be used to generate large peptide libraries which can be screened for peptides with desired binding characteristics as described in US 5223409 to Ladner et al. and US 5571698 to Ladner et al. In the two latter patents, a phage based system is used in which each randomized peptide is fused to the gene III protein of the M13 phage. However, also non-phage based systems such as screening peptides on polysomes or on the surface of *E. coli* (Tuerk *&* Gold, 1990, Science 249:505-510), or, using a system wherein each randomized peptide is fused to a DNA binding protein as described in US 5498530 to Schatz: et al. can be used.

The scaffold of the present invention carrying randomized aa sequences can be used for therapeutic, diagnostic, and related purposes. The man skilled in the art will appreciate that the targets or ligands bound by the recombinant proteins of the present invention can be proteins, nucleic acids, lipids and carbohydrates, or combinations thereof It has to be understood that 'combinations thereof' refers to glycoproteins, lipoproteins, and the like but also refers to dimeric (homo- or heterodimeric) biomolecules that are bound to each other by non-covalent means. Therefore, the term 'antigen' as used throughout the specification, has to be interpreted in the broadest sense. Because one can use an artificial screening system, the man skilled in the art will also appreciate that recombinant proteins can also be selected or screened for binding to xenobiotic molecules, that would otherwise be highly toxic for a biological system.

It has to be understood that the invention also relates to those recombinant proteins that are able to bind, as already indicated above, two entirely unrelated biomolecules, by means of two binding surfaces, e.g. one surface consisting of the loops connecting the β-strands on one side of the β-sandwich and comprising the CDR loops in the natural protein, and one surface consisting of the loops that connect the β-strands on the other side of the β-sandwich. As such, both binding surfaces can operate independent from each other and selection or screening for such proteins can be achieved in two separate rounds:
- in the first round, the biomolecule that binds to one partner is retrieved from a library wherein the amino acid sequence of one set of loops has been randomized while the other set of loops is untouched e.g. as in the naturally occurring protein,
- in the second round, the biomolecule that binds to a second partner is retrieved from a library wherein the amino acid sequence of the other set of loops has been randomized. In the second round, the randomized library may already contain those loops that bind for the first partner or, alternatively, remain untouched as in the naturally occurring protein. In the latter case, both sets of binding loops as retrieved which bind separate partners have to be recombined within a single scaffolding protein. One binding partner can be used to target for certain cell types such as cancer cells inducing intracellular uptake, while the other binding partner can be a component of a life sustaining cellular mechanism of which the function is inhibited upon binding. One of the binding sites can also be a cytotoxic molecule, thereby forcing uptake of the cytotoxic molecule into the targeted cells. This strategy can also be used to force stake of endogeneous molecules, such as auto-immune antibodies, toward cell types, such as liver cells. One binding partner can be used to target for certain cell types, while the other binding partner can be used to target for other cell types thereby forcing both cell types to stay in close proximity, such as any lymphocytes with for instance lymphocytes of a different type or with instance cancer cells. One of the binding partners can also be used to function as a spacer in cases wherein immobilisation of the entire protein is desired, such as on membranes or on any carrier for chromatographic purposes.

The present invention also regards, as stated above, a scaffold as defined above wherein said single-chain polypeptide is unglycosylated. However, it has to be understood that the scaffold of the present invention can also be glycosylated. Though one of the major advantages of the claimed scaffolds is the fact that they remain functional without glycosylation, allowing expression in bacterial systems, the fact of being unglycosylated is not a prerequisite for falling under the protected matter. Once a recombinant scaffold has been selected in a bacterial background for binding a specific molecule and thus as an unglycosylated protein, one can decide to glycosylate the scaffold by expression in a eukaryotic background. This can have distinct advantages over the nonglycosylated scaffold for therapeutic purposes.

The present invention will now be illustrated by reference to the following examples which set forth particularly advantageous embodiments. However, it should be noted that these embodiments are illustrative and can not be construed as to restrict the invention in any way.

### EXAMPLES

### Example 1: Cloning of a recombinant scaffold, named SCA, for display on the surface of filamentous phage

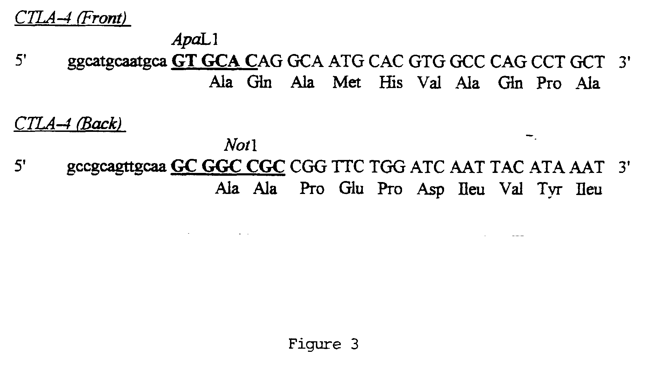

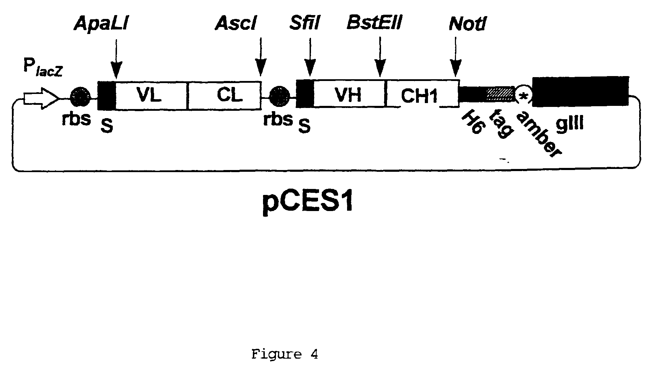

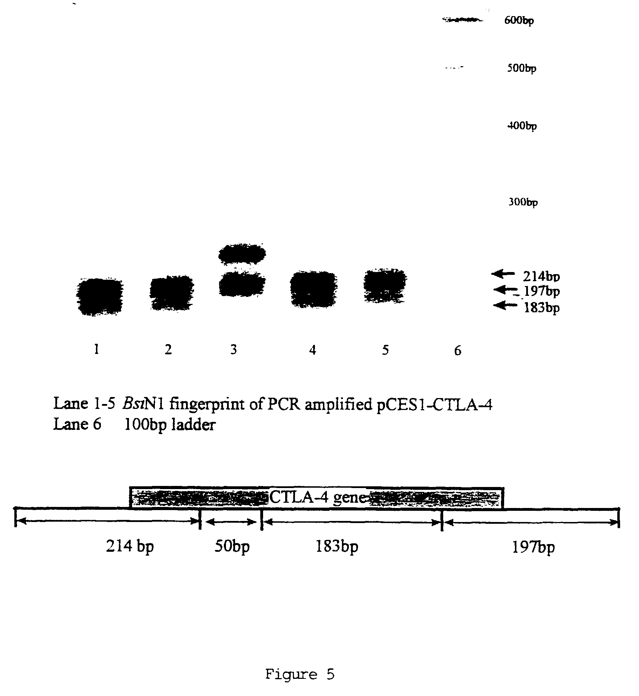

The sequence coding for SCA, which is based on the extracellular part of human CTLA-4 gene as described by Metzler *et al.* (1997) but without the C-terminal cysteine residue (Cys 123), is cloned into the phage display vector pCES-1 (figure 4). The cysteine residue is not included because it my present some problems with the correct folding and presentation of the SCA- p3 fusion on filamentous phage. This cloning creates a translational fusion of SCA to the N-terminus of p3 for display on filamentous phage. SCA is cloned as a *Apa*L1/*Not*1 fragment (neither of these restriction sites are present in human CTLA-4) using specific primers (figure 3). Human CTLA-4 is amplified from 0.1ng vector pBSK(+)hCTLA-4 using primers CTLA-4 (Front) and CTLA-4 (Back) at 10pM, 10µl PCR buffer plus magnesium at 2.5mM, 5µl of dNTP's and 1µl of taq polymerase (Boerhinger Manheim EXPAND™). Amplification is done with a hot start of 10 minutes at 94°C and then for 30 cycles 1 min 94°C, 1 min 50°C and 2 min 72°C. The correct sized PCR product is then purified (402bp) and digested with both *Apa*L1 and *Not*1. Vector pCES1 is also digested with *Apa*L1 and *Not*1. Both digested vector and digested SCA- insert are purified. Ligation is performed overnight at 16°C and is transformed into TG1. The *Bst*N1 fingerprints of clones with the correct sized insert, as assessed by PCR, demonstrate the expected patterns (Figure 5).

### Example 2: Display of a recombinant, non-glycosylated and monomeric SCA-protein on the surface of filamentous phage

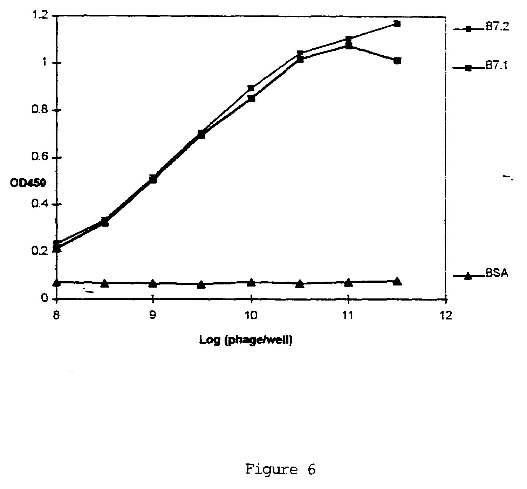

Phage displaying non-glycosylated SCA proteins on their surface are prepared as follows. Clones 2, 4 and 5 from figure 4 are grown to an OD of 0.5 in the presence of ampicillin at 100µg/ml and 2% glucose. Five mls of culture is infected with helper phage M13KO7 at a multiplicity of infection of 20:1 and incubated at 37°C for 30 minutes. Cells are resuspended in TY medium plus Amplicillin and Kanamycin (25µg/ml) and grown overnight at 30°C. Phages are recovered by precipitation by PEG and resuspended in PBS. Because the SCA protein is derived from the human CTLA-4 protein wherein the original CDR loops are retained, a binding is expected with B7.1 and B7.2 proteins. To demonstrate that such binding occurs, phages are titrated and a serial dilution is made to test for binding in ELISA to B7.1-Ig and B7.2-Ig. A strong signal is obtained showing binding to both B7.1-Ig and B7.2-Ig and not to BSA or plastic (Figure 6). The latter signal decreases proportionately with the amount of phage present. This demonstrates that un-glycosylated SCA has the proper conformation and remains functional as a monomer. There is no difference in binding behavior for B7-1 or B7-2 of SCA displaying phage.

### Example 3: Soluble SCA - His6/myc expressed in the non-suppressor strain HB2151

The gene coding for SCA, preceded by the pelB signal sequence, is cloned into the phage display vector pCES-1 (figure 4) under control of the IPTG inducible lac-promoter. The coding sequence is then fused C-terminally to a His6 followed with a myc-tag. An amber stopcodon is located at the junction between this coding sequence and the sequence coding for the phage coat protein g3p. This allows one to choose whether the protein will be exposed on the surface of the viral coat through fusion with the g3p coat protein, or expressed as a soluble protein, without the need for a supplementary cloning step. When a suppressor type of strain is transformed with this construct, translation will continue through the stopcodon resulting in a fused SCA-g3p protein exposed on the surface of a tip of the phage. In a non-suppressor strain the amber stopcodon is recognized and translation stops. Subsequently, the His6- or myc-tagged protein is transported through the cell membrane and accumulates in the periplasm. Non-suppressor cells HB2151 are transformed with the His6/myc- tagged construct and grown in liquid LB medium with ampicilline for selection and 1% glucose for metabolic repression of the lac-promoter at 28 °C until saturation is reached. This saturated culture is subsequently diluted 20 times in 20 ml LB+A+G and grown at 28 °C until an OD600 is reached of 0.5 to 0.6, which takes about three hours. The glucose containing medium is then removed and replaced by LB+A ad 0. 1 mM IPTG. The induced culture is further incubated for about 20 hours. The next day the OD600 is measured and the cells are fractionated.

### Example 4: Fractionation of transformed cells

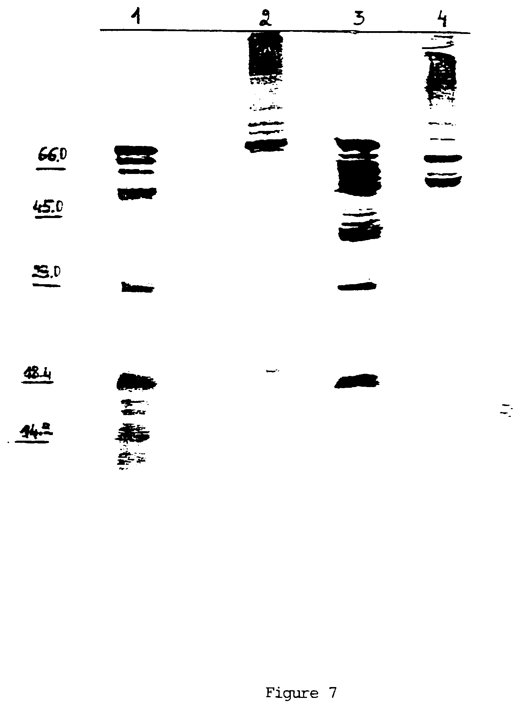

To examine whether the expressed SCA protein is actually secreted and accumulated in the periplasm, and possibly leaking out into the medium, a periplasmic fraction is prepared using a modified protocol as according to the osmotic shock procedure described by Neu and Heppel (1965).
Sixteen ml of cell culture is centrifuged en resuspended in 1 ml of icecold TES-buffer (200 mM Tris pH8, 20 % sucrose; 50 mM EDTA). The cells are then incubated for 10 min on ice and vortexed regularly. Subsequently, the mixture is centrifuged at 10,000 rpm for 1.5 min and the supernatant is disgarded. The pellet is quickly taken up in 1 ml ice cold distilled water. After 10 min on ice and regular vortexing the mixture is centrifuged at 14,000 rpm for 2 min and the resulting supernatant is recuperated as a periplasmic fraction. This material was compared to pelleted cells and material leaking into the medium, by means of SDS-page and detection with anti-His or anti-myc. This demonstrates that part of the expressed 16 kD SCA protein is indeed present in the periplasm and that part is leaking out into the medium (results not shown). The material present in the medium was dialysed against PBS and analyzed by means of SDS-page and detection with anti-CTLA4 or anti -myc (see figure 7). The 16 kD SCA-protein is clearly present. The 83 kD band is probably due to a fusion of the SCA protein with the gene 3 coat protein of the phage.

### Example 5: The soluble, bacterially expressed, non-glycosylated SCA-molecule binds B7.1 and B7.2 on the membrane

The soluble SCA-molecule is tested for binding capacity to the human B7.1 and B7.2 molecules. The EBV-transformed B cell line (RPMI 8866, 0.5-1x 10⁵ cells/sample), which strongly expresses B7.1 and B7.2, is incubated for 20 min at 4°C with the soluble SCA-molecule. After washing twice in RPMI 1640 supplemented with 10% FCS, the cells are incubated for another 20 min at 4°C with mouse anti-cMyc mAb (1µg/cell pellet) conjugated to biotine. After washing twice in RPMI 1640 supplemented with 10% FCS, the cells are incubated for another 20 min at 4°C with Streptavidin conjugated to PE (Phycoerythrine). The cells are then washed twice in RPMI 1640 supplemented with 10% FCS and finally suspended in PBS supplemented with 1% BSA and 0.1% NaN₃ and analyzed with a FACScan flow cytometer (Becton Dickinson). The specific binding of the SCA-molecule is expressed as the mean fluorescent intensity in arbitrary units. The results show that soluble, non-glycosylated SCA binds, in a dose-dependent way, to the RPMI 8866 cells which express B7.1 and B7.2 (results not shown).

### LIST OF REFERENCES

- **Amit,A.G., Mariuzza,R.A.,Phillips,S.E.V., and Poljak,R.J.** (1986) Three dimensional structure of an antigen -antibody coplex at 2.8Å resolution. Science 233: 747
- **Amzel,L.M. and Poljak,R.J.** (1979) Three dimensional structure of immunoglobulins. Ann.Rev.Biochem 48: 961
- **Atherton & Shepard** (1989) Solid phase peptide synthesis. IRL Press, Oxford.
- **Bianchi,E.,Sollazzo,M.,Tramontano,A.,Pessi,A.** (1993) Affinity purification of a difficult sequence protein. Int.J.Peptide Protein Res. 42: 93
- **Bianchi,E,Venturini,S.,Pessi,A.,Tramontano,A.,Sollazzo,M.** (1994) High level expression and rational mutagenesis of a designed protein, the minibody: from an insoluble to a soluble protein.
- **Cannon E.L., Ladner R.C., McCoy D.** (1996) Phage-display technology. IVD Technology Nov./Dec., Canon Communications.
- **Houbenweyl** (1974) Methode der organischen chemie, vol. 15, I & II (ed. Wunch E). Thieme, Stuttgart.
- **Kubinyi H.** (1995). Strategies and recent technologies in drug discovery. Pharmazia 50:647-662.
- **Ladner R.** (1995) Constrained peptides as binding entities.TibTech 13:426-430.
- **Maclennan J.** (1995) Engineering microprotein ligands for large-scale affinity purification. Biotechnology 13: 1181-1183.
- **Maniatis T., Fritsch E., Sambrook J.** (1982) Molecular cloning: a laboratory manual. Cold Spring Harbor Laboratory Press, Cold Spring Harbor, NY.
- **Metzler W.J., Bajorath J., Fenderson W., Shaw S., Constantine K., Naemura J., Leytze G., Peach R., Lavoie T., Mueller L., Linsley P.** (1997) Solution structure of human CTLA-4 and delineation of a CD80/CD86 binding site conserved in CD28. Nat. Struct. Biol. 4: 527-531.
- **Neu,H.C. and Heppel,L.A.** (1965) J.Biol.Chem 240: 3685
- **Reiter,Y.,Brinkmann,U.,Webber,K.,Jung,S.-H.,Lee,B.K. and Pastan,I** (1994) Engineering interchain disulfide bonds into conserved framework regions of Fv fragments: improved biochemical characteristics of recombinant immunotoxins containing disulfide-stabilized Fv. Protein Eng. 7: 697
- **Raag,R., and Whitlow,M.** (1995) Single-chain Fvs. FASEB J. 9:73
- **Williams A.F.** (1987) A year in the life of the immunoglobulin superfamily. Immunol.Today 8: 298

## Claims

1. A scaffold composed of a single-chain polypeptide having the following structural properties:
- it contains at least two cysteine residues which form at least one disulphide bond, and
- it possesses less than 10% of alpha helical conformation, and
- it contains at least six β-strands (S1, S2, S3, S4, S5, S6) which:
- are connected by amino acid loops of variable conformation and lenght according to the following topology: S1-S2-S3-S4-S5-S6, and
- form two β-sheets, one formed by S1/S4/S3 and one formed by S6/S5/S2 wherein the symbol "/" denotes the hydrogen bonding interactions between two spatially adjacent β strands, which are each characterized by an anti-parallel arrangement of said β strands and which are packed onto eachother so that they form a β sandwich architecture.

2. A scaffold according to claim 1 which:
- contains maximum three additional β strands (A1, A2 and A3), and
- has the following said topology: A1-S1-S2-A2-A3-S3-S4-S5-S6, and
- possesses the following said β-sheets: A1/S1/S4/S3 and S6/S5/S2/A2/A3.

3. A scaffold according to claims 1 and 2 wherein:
- A1 is the amino acid sequence AQPAVVLA (SEQ ID 1) or any functionally equivalent derivative of said sequence,
- S1 is the amino acid sequence ASFPVEY (SEQ ID 2) or any functionally equivalent derivative of said sequence,
- S2 is the amino acid sequence EVRVTVLRQA (SEQ ID 3) or any functionally equivalent derivative of said sequence,
- A2 is the amino acid sequence QVTEVCAA (SEQ ID 4) or any functionally equivalent derivative of said sequence,
- A3 is the amino acid sequence TYMMGNELTFLDDS (SEP ID 5) or any functionally equivalent derivative of said sequence,
- S3 is the amino acid sequence ICTGTSS (SEQ ID 6) or any functionally equivalent derivative of said sequence,
- S4 is the amino acid sequence QVNLTIQ (SEQ ID 7) or any functionally equivalent derivative of said sequence,
- S5 is the amino acid sequence GLYICKVE( SEQ ID 8) or any functionally equivalent derivative of said sequence,
- S6 is the amino acid sequence GIGNGTQIY (SEQ ID 9) or any functionally equivalent derivative of said sequence.

4. A scaffold according to claims 1 to 3 wherein said single-chain polypeptide is unglycosylated.

5. A scaffold according to claims 1 to 4 wherein said amino acid loops comprise fragments binding to a receptor or antigen.

6. A scaffold according to claim 4 wherein said amino acid loops comprise fragments binding B7.1 and/or B7.2.

7. A single-chain polypeptide as defined in claims 1 to 6 for use as a scaffold.


SEQUENCE LISTING

(1) GENERAL INFORMATION:

(i) APPLICANT:
(A) NAME: INNOGENETICS NV
(B) STREET: INDUSTRIEPARK ZWIJNAARDE NR 7, BOX 4
(C) CITY: GHENT
(E) COUNTRY: BELGIUM
(F) POSTAL CODE (ZIP): B9052
(G) TELEPHONE: 32 9 241 07 11
(H) TELEFAX: 32 9 241 07 99

(ii) TITLE OF INVENTION: A polypeptide structure for use as a
scaffold.

(iii) NUMBER OF SEQUENCES: 236

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98870065.4

(2) INFORMATION FOR SEQ ID NO: 1:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 1:

Ala Gln Pro Ala Val Val Leu Ala
1               5

(2) INFORMATION FOR SEQ ID NO: 2:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 2:

Ala Ser Phe Pro Val Glu Tyr
1               5

(2) INFORMATION FOR SEQ ID NO: 3:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 3:

Glu Val Arg Val Thr Val Leu Arg Gln Ala
1               5                   10

(2) INFORMATION FOR SEQ ID NO: 4:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 4:

Gln Val Thr Glu Val Cys Ala Ala
1               5

(2) INFORMATION FOR SEQ ID NO: 5:

(i) SEQUENCE CHARACTERISTICS:

(A) LENGTH: 14 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 5:

Thr Tyr Met Met Gly Asn Glu Leu Thr Phe Leu Asp Asp Ser
1               5                   10

(2) INFORMATION FOR SEQ ID NO: 6:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 6:

Ile Cys Thr Gly Thr Ser Ser
1               5

(2) INFORMATION FOR SEQ ID NO: 7:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 7:

Gln Val Asn Leu Thr Ile Gln
1               5

(2) INFORMATION FOR SEQ ID NO: 8:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 8:

Gly Leu Tyr Ile Cys Lys Val Glu
1               5

(2) INFORMATION FOR SEQ ID NO: 9:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 9:

Gly Ile Gly Asn Gly Thr Gln Ile Tyr
1               5

(2) INFORMATION FOR SEQ ID NO: 10:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 10:

Thr Gln Pro Ser Val Val Leu Ala
1               5

(2) INFORMATION FOR SEQ ID NO: 11:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 11:

Thr Gln Pro Pro Val Val Leu Ala
1               5

(2) INFORMATION FOR SEQ ID NO: 12:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 12:

Ser Gln Pro Ala Val Val Leu Ala
1               5

(2) INFORMATION FOR SEQ ID NO: 13:

(i) SEQUENCE CHARACTERISTICS:
(A) LENGTH: 9 amino acids
(B) TYPE: amino acid

(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 13:

Lys Gln Ser Pro Leu Leu Val Val Asp
1               5

(2) INFORMATION FOR SEQ ID NO: 14:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 14:

Lys Gln Ser Pro Met Leu Val Val Asn
1               5

(2) INFORMATION FOR SEQ ID NO: 15:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 15:

Lys Gln Ser Pro Met Leu Val Ala Tyr

1                   5

(2) INFORMATION FOR SEQ ID NO: 16:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 16:

Lys Gln Leu Pro Arg Leu Val Val Tyr
1               5

(2) INFORMATION FOR SEQ ID NO: 17:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 17:

Ala Gln Arg Pro Leu Leu Ile Val Ala
1               5

(2) INFORMATION FOR SEQ ID NO: 18:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 18:

Thr Gln Ser Pro Ala Ile Met Ser Ala
1               5

(2) INFORMATION FOR SEQ ID NO: 19:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 19:

Gln Glu Ser Gly Pro Gly Leu Val
1               5

(2) INFORMATION FOR SEQ ID NO: 20:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 20:

Ala Ser Phe Pro Cys Glu Tyr
1               5

(2) INFORMATION FOR SEQ ID NO: 21:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 21:

Ala Ser Phe Ser Cys Glu Tyr
1               5

(2) INFORMATION FOR SEQ ID NO: 22:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 22:

Val Ser Leu Ser Cys Arg Tyr
1               5

(2) INFORMATION FOR SEQ ID NO: 23:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 23:

Val Asn Leu Ser Cys Lys Tyr
1               5

(2) INFORMATION FOR SEQ ID NO: 24:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 24:

Ala Thr Leu Val Cys Asn Tyr
1               5

(2) INFORMATION FOR SEQ ID NO: 25:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 25:

Val Thr Met Thr Cys Ser Ala
1               5

(2) INFORMATION FOR SEQ ID NO: 26:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 26:

Val Thr Met Thr Cys Lys Ser
1               5

(2) INFORMATION FOR SEQ ID NO: 27:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 27:

Val Thr Ile Thr Cys Lys Ala
1               5

(2) INFORMATION FOR SEQ ID NO: 28:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 28:

Val Thr Met Ser Cys Lys Ser
1               5

(2) INFORMATION FOR SEQ ID NO: 29:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 29:

Met Lys Leu Ser Cys Val Ala
1               5

(2) INFORMATION FOR SEQ ID NO: 30:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 30:

Leu Arg Leu Ser Cys Ala Thr
1               5

(2) INFORMATION FOR SEQ ID NO: 31:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 31:

Leu Lys Leu Ser Cys Ala Ala
1               5

(2) INFORMATION FOR SEQ ID NO: 32:

(i) SEQUENCE CHARACTERISTICS:

(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 32:

Arg Lys Leu Ser Cys Ala Ala
1               5

(2) INFORMATION FOR SEQ ID NO: 33:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 33:

Leu Ser Ile Thr Cys Thr Val
1               5

(2) INFORMATION FOR SEQ ID NO: 34:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 34:

Val Lys Leu Ser Cys Thr Ala
1               5

(2) INFORMATION FOR SEQ ID NO: 35:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 35:

Val Lys Ile Ser Cys Lys Ala
1               5

(2) INFORMATION FOR SEQ ID NO: 36:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 36:

Leu Ser Ile Thr Cys Thr Val
1               5

(2) INFORMATION FOR SEQ ID NO: 37:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 37:

Val Lys Ile Ser Cys Lys Ala
1               5

(2) INFORMATION FOR SEQ ID NO: 38:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 38:

Val Gln Ile Ser Cys Lys Ala
1               5

(2) INFORMATION FOR SEQ ID NO: 39:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 39:

Glu Val Arg Val Thr Val Leu Arg Gln Thr
1               5                   10

(2) INFORMATION FOR SEQ ID NO: 40:

(i) SEQUENCE CHARACTERISTICS:
(A) LENGTH: 10 amino acids
(B) TYPE: amino acid

(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 40:

Glu Val Arg Val Thr Val Leu Arg Glu Ala
1               5                   10

(2) INFORMATION FOR SEQ ID NO: 41:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 41:

Glu Phe Arg Ala Ser Leu Tyr Lys Gly Val
1               5                   10

(2) INFORMATION FOR SEQ ID NO: 42:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 42:

Glu Phe Arg Ala Ser Leu Tyr Lys Gly Ala

1                   5                   10

(2) INFORMATION FOR SEQ ID NO: 43:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 43:

Glu Phe Arg Ala Ser Leu His Lys Gly Leu
1               5                   10

(2) INFORMATION FOR SEQ ID NO: 44:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 44:

Glu Phe Arg Ala Ser Leu His Lys Gly Thr
1               5                   10

(2) INFORMATION FOR SEQ ID NO: 45:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 45:

Glu Val Arg Met Tyr Met Tyr Gln Gln Lys
1               5                   10

(2) INFORMATION FOR SEQ ID NO: 46:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 46:

Lys Asn Tyr Leu Thr Trp Tyr Gln Gln Lys
1               5                   10

(2) INFORMATION FOR SEQ ID NO: 47:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 47:

Glu Val Arg Val Val Trp Tyr Gln Gln Lys
1               5                   10

(2) INFORMATION FOR SEQ ID NO: 48:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 48:

Asn Phe Arg Leu Ala Trp Tyr Gln Gln Lys
1               5                   10

(2) INFORMATION FOR SEQ ID NO: 49:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 49:

Lys Asn Phe Leu Ala Trp Tyr Gln Gln Lys
1               5                   10

(2) INFORMATION FOR SEQ ID NO: 50:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 50:

Glu Val Arg Met Ser Trp Val Arg Gln Ser
1               5                   10

(2) INFORMATION FOR SEQ ID NO: 51:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 51:

Glu Val Arg Met Glu Trp Val Arg Gln Pro
1               5                   10

(2) INFORMATION FOR SEQ ID NO: 52:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 52:

Glu Val Arg Met Ser Trp Val Arg His Thr
1               5                   10

(2) INFORMATION FOR SEQ ID NO: 53:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 53:

Glu Val Arg Met His Trp Val Arg Gln Ala
1               5                   10

(2) INFORMATION FOR SEQ ID NO: 54:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 54:

Glu Val Arg Val Asn Trp Val Arg Gln Pro
1               5                   10

(2) INFORMATION FOR SEQ ID NO: 55:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 55:

Glu Val Arg Met Asn Trp Val Lys Gln Arg
1               5                   10

(2) INFORMATION FOR SEQ ID NO: 56:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 56:

Glu Val Arg Ile Glu Trp Val Lys Gln Arg
1               5                   10

(2) INFORMATION FOR SEQ ID NO: 57:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 57:

Glu Val Arg Val His Trp Val Arg Gln Ser
1               5                   10

(2) INFORMATION FOR SEQ ID NO: 58:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 58:

Glu Val Arg Ile Glu Trp Val Lys Glu Arg
1               5                   10

(2) INFORMATION FOR SEQ ID NO: 59:

(i) SEQUENCE CHARACTERISTICS:

(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 59:

Gln Val Thr Glu Val Cys Ala Thr
1               5

(2) INFORMATION FOR SEQ ID NO: 60:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 60:

Gln Met Thr Glu Val Cys Ala Thr
1               5

(2) INFORMATION FOR SEQ ID NO: 61:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 61:

Gln Val Thr Glu Val Cys Ala Gly
1               5

(2) INFORMATION FOR SEQ ID NO: 62:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 62:

Gln Met Thr Glu Val Cys Ala Met
1               5

(2) INFORMATION FOR SEQ ID NO: 63:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 63:

Ser Asp Val Glu Val Cys Val Gly
1               5

(2) INFORMATION FOR SEQ ID NO: 64:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 64:

Ser Ala Val Glu Val Cys Val Val
1               5

(2) INFORMATION FOR SEQ ID NO: 65:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 65:

Ser Ala Val Glu Val Cys Ala Val
1               5

(2) INFORMATION FOR SEQ ID NO: 66:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 66:

Ser Ala Val Glu Val Cys Phe Ile
1               5

(2) INFORMATION FOR SEQ ID NO: 67:

(i) SEQUENCE CHARACTERISTICS:
(A) LENGTH: 8 amino acids
(B) TYPE: amino acid

(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 67:

Ser Pro Arg Leu Leu Ile Tyr Asp
1               5

(2) INFORMATION FOR SEQ ID NO: 68:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 68:

Ser Pro Lys Leu Leu Ile Tyr Trp
1               5

(2) INFORMATION FOR SEQ ID NO: 69:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 69:

Pro Pro Lys Leu Leu Ile Tyr Gly

1               5

(2) INFORMATION FOR SEQ ID NO: 70:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 70:

Gly Leu Glu Trp Val Ala Glu Ile
1               5

(2) INFORMATION FOR SEQ ID NO: 71:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 71:

Arg Leu Glu Trp Ile Ala Ala Ser
1               5

(2) INFORMATION FOR SEQ ID NO: 72:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 72:

Arg Leu Glu Trp Val Ala Thr Ile
1               5

(2) INFORMATION FOR SEQ ID NO: 73:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 73:

Gly Leu Glu Trp Val Ala Tyr Ile
1               5

(2) INFORMATION FOR SEQ ID NO: 74:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 74:

Gly Leu Glu Trp Leu Gly Met Ile
1               5

(2) INFORMATION FOR SEQ ID NO: 75:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 75:

Gly Leu Glu Trp Ile Gly Arg Ile
1               5

(2) INFORMATION FOR SEQ ID NO: 76:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 76:

Gly Leu Glu Trp Ile Gly Trp Ile
1               5

(2) INFORMATION FOR SEQ ID NO: 77:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 77:

Gly Leu Glu Trp Leu Gly Met Ile
1               5

(2) INFORMATION FOR SEQ ID NO: 78:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 78:

Gly Leu Glu Trp Ile Gly Glu Ile
1               5

(2) INFORMATION FOR SEQ ID NO: 79:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 79:

Gly Leu Glu Trp Ile Gly Glu Ile
1               5

(2) INFORMATION FOR SEQ ID NO: 80:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 80:

Thr Phe Thr Val Lys Asn Thr Leu Gly Phe Leu Asp Asp Pro
1               5                   10

(2) INFORMATION FOR SEQ ID NO: 81:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 81:

Thr Phe Thr Glu Lys Asn Thr Val Gly Phe Leu Asp Tyr Pro
1               5                   10

(2) INFORMATION FOR SEQ ID NO: 82:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 82:

Thr Tyr Met Val Glu Asp Glu Leu Thr Phe Leu Asp Asp Ser
1               5                   10

(2) INFORMATION FOR SEQ ID NO: 83:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 83:

Thr Tyr Thr Val Glu Asn Glu Leu Thr Phe Ile Asp Asp Ser
1               5                   10

(2) INFORMATION FOR SEQ ID NO: 84:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 84:

Asn Gly Asn Phe Thr Tyr Gln Pro Gln Phe Arg Ser Asn Ala Glu Phe
1               5                   10                  15

(2) INFORMATION FOR SEQ ID NO: 85:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 85:

Asn Gly Asn Phe Thr Tyr Gln Pro Gln Phe Arg Pro Asn Val Gly Phe
1               5                   10                  15

(2) INFORMATION FOR SEQ ID NO: 86:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 86:

Asn Gly Asn Phe Ser His Pro His Gln Phe His Ser Thr Thr Gly Phe
1               5                   10                  15

(2) INFORMATION FOR SEQ ID NO: 87:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 87:

Asn Gly Asn His Ser His Pro Leu Gln Ser His Thr Asn Lys Glu Phe
1               5                   10                  15

(2) INFORMATION FOR SEQ ID NO: 88:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 88:

Tyr Gly Asn Tyr Ser Gln Gln Leu Gln Val Tyr Ser Lys Thr Gly Phe
1               5                   10                  15

(2) INFORMATION FOR SEQ ID NO: 89:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 89:

Asn Gly Asn Tyr Ser His Gln Pro Gln Phe Tyr Ser Ser Thr Gly Phe
1               5                   10                  15

(2) INFORMATION FOR SEQ ID NO: 90:

(i) SEQUENCE CHARACTERISTICS:
(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

. (xi) SEQUENCE DESCRIPTION: SEQ ID NO: 90:

Ser Trp Asn Met Thr His Lys Ile Asn Ser Asn Ser Asn Lys Glu Phe
1               5                   10                  15

(2) INFORMATION FOR SEQ ID NO: 91:

(i) SEQUENCE CHARACTERISTICS:
(A) LENGTH: 10 amino acids
(B) TYPE: amino acid
(C) STRANDEDNESS: single

(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 91:

Thr Ser Asn Leu Ala Ser Gly Val Pro Val
1               5                   10

(2) INFORMATION FOR SEQ ID NO: 92:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 92:

Ala Ser Thr Arg Glu Ser Gly Val Pro Asp
1               5                   10

(2) INFORMATION FOR SEQ ID NO: 93:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 93:

Ala Ser Thr Arg His Ile Gly Val Pro Asp
1               5                   10

(2) INFORMATION FOR SEQ ID NO: 94:

(i) SEQUENCE CHARACTERISTICS:
(A) LENGTH: 17 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 94:

Arg Leu Asn Ser Asp Asn Phe Ala Thr His Tyr Ala Glu Ser Val Lys
1               5                   10                  15

Gly

(2) INFORMATION FOR SEQ ID NO: 95:

(i) SEQUENCE CHARACTERISTICS:
(A) LENGTH: 17 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 95:

Arg Asn Lys Gly Asn Lys Tyr Thr Thr Glu Tyr Ser Ala Ser Val Lys
1               5                   10                  15

Gly

(2) INFORMATION FOR SEQ ID NO: 96:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 96:

Ser Asn Gly Gly Gly Tyr Thr Tyr Tyr Gln Asp Ser Val Lys Gly
1               5                   10                  15

(2) INFORMATION FOR SEQ ID NO: 97:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 97:

Ser Ser Gly Ser Ser Thr Leu His Tyr Ala Asp Thr Val Lys Gly
1               5                   10                  15

(2) INFORMATION FOR SEQ ID NO: 98:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 98:

Trp Gly Asp Gly Asn Thr Asp Tyr Asn Ser Ala Leu Lys Ser
1               5                   10

(2) INFORMATION FOR SEQ ID NO: 99:

(i) SEQUENCE CHARACTERISTICS:

(A) LENGTH: 15 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 99:

Asp Pro Ala Asn Gly Asn Ile Gln Tyr Asp Pro Lys Phe Arg Gly
1               5                   10                  15

(2) INFORMATION FOR SEQ ID NO: 100:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 100:

Tyr Pro Gly Ser Gly Asn Thr Lys Tyr Asn Glu Lys Phe Lys Gly
1               5                   10                  15

(2) INFORMATION FOR SEQ ID NO: 101:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 101:

Leu Pro Gly Ser Gly Ser Thr Asn Tyr Asn Glu Lys Phe Lys Gly
1               5                   10                  15

(2) INFORMATION FOR SEQ ID NO: 102:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 102:

Trp Gly Gly Gly Ser Ile Glu Tyr Asn Pro Ala Leu Lys Ser
1               5                   10

(2) INFORMATION FOR SEQ ID NO: 103:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 103:

Leu Pro Gly Ser Gly Arg Thr Asn Tyr Arg Glu Lys Phe Lys Gly
1               5                   10                  15

(2) INFORMATION FOR SEQ ID NO: 104:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 104:

Phe Cys Ser Gly Thr Phe Asn
1               5

(2) INFORMATION FOR SEQ ID NO: 105:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 105:

Thr Cys Ile Gly Thr Ser Arg
1               5

(2) INFORMATION FOR SEQ ID NO: 106:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 106:

Thr Cys Thr Gly Ile Ser His
1               5

(2) INFORMATION FOR SEQ ID NO: 107:

(i) SEQUENCE CHARACTERISTICS:
(A) LENGTH: 7 amino acids
(B) TYPE: amino acid

(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 107:

Asn Cys Asp Gly Asp Phe Asp
1               5

(2) INFORMATION FOR SEQ ID NO: 108:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 108:

Asn Cys Asp Gly Asn Phe Asp
1               5

(2) INFORMATION FOR SEQ ID NO: 109:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 109:

Asn Cys Thr Val Lys Val Gly

1               5

(2) INFORMATION FOR SEQ ID NO: 110:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 110:

Asn Cys Thr Val Lys Val Gly
1               5

(2) INFORMATION FOR SEQ ID NO: 111:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 111:

Asn Cys Asp Gly Lys Leu Gly
1               5

(2) INFORMATION FOR SEQ ID NO: 112:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 112:

Asp Cys Asp Gly Lys Leu Gly
1               5

(2) INFORMATION FOR SEQ ID NO: 113:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 113:

Asn Cys Arg Gly Ile His Asp
1               5

(2) INFORMATION FOR SEQ ID NO: 114:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 114:

Arg Phe Ser Gly Ser Gly Ser
1               5

(2) INFORMATION FOR SEQ ID NO: 115:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 115:

Arg Phe Thr Gly Ser Gly Ser
1               5

(2) INFORMATION FOR SEQ ID NO: 116:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 116:

Arg Phe Ala Gly Ser Gly Ser
1               5

(2) INFORMATION FOR SEQ ID NO: 117:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 117:

Lys Phe Ile Ile Ser Arg Asp
1               5

(2) INFORMATION FOR SEQ ID NO: 118:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 118:

Arg Phe Ile Val Ser Arg Asp
1               5

(2) INFORMATION FOR SEQ ID NO: 119:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 119:

Arg Phe Thr Ile Ser Arg Asp
1               5

(2) INFORMATION FOR SEQ ID NO: 120:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 120:

Arg Phe Thr Ile Ser Arg Asp
1               5

(2) INFORMATION FOR SEQ ID NO: 121:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 121:

Arg Leu Ser Ile Ser Phe Asp
1               5

(2) INFORMATION FOR SEQ ID NO: 122:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 122:

Lys Ala Thr Ile Thr Ala Asp
1               5

(2) INFORMATION FOR SEQ ID NO: 123:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 123:

Lys Ala Thr Leu Thr Val Asp
1               5

(2) INFORMATION FOR SEQ ID NO: 124:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 124:

Arg Leu Ser Ile Ser Lys Asp
1               5

(2) INFORMATION FOR SEQ ID NO: 125:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 125:

Lys Ala Thr Phe Thr Ala Asp
1               5

(2) INFORMATION FOR SEQ ID NO: 126:

(i) SEQUENCE CHARACTERISTICS:

(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 126:

Arg Val Asn Leu Thr Ile Gln
1               5

(2) INFORMATION FOR SEQ ID NO: 127:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 127:

Lys Val Asn Leu Thr Ile Gln
1               5

(2) INFORMATION FOR SEQ ID NO: 128:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 128:

Thr Val Thr Phe Arg Leu Trp
1               5

(2) INFORMATION FOR SEQ ID NO: 129:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 129:

Thr Val Thr Phe Tyr Leu Lys
1               5

(2) INFORMATION FOR SEQ ID NO: 130:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 130:

Thr Val Thr Phe Tyr Leu Gln
1               5

(2) INFORMATION FOR SEQ ID NO: 131:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 131:

Ser Val Thr Phe Tyr Leu Gln
1               5

(2) INFORMATION FOR SEQ ID NO: 132:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 132:

Thr Val Thr Phe Tyr Leu Arg
1               5

(2) INFORMATION FOR SEQ ID NO: 133:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 133:

Lys Val Ile Phe Asn Leu Trp
1               5

(2) INFORMATION FOR SEQ ID NO: 134:

(i) SEQUENCE CHARACTERISTICS:
(A) LENGTH: 7 amino acids
(B) TYPE: amino acid

(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 134:

Ser Tyr Ser Leu Thr Ile Ser
1               5

(2) INFORMATION FOR SEQ ID NO: 135:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 135:

Asp Phe Thr Leu Ser Ile Ser
1               5

(2) INFORMATION FOR SEQ ID NO: 136:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 136:

Asp Tyr Thr Leu Thr Ile Ser

1               5

(2) INFORMATION FOR SEQ ID NO: 137:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 137:

Asp Phe Thr Leu Thr Ile Ser
1               5

(2) INFORMATION FOR SEQ ID NO: 138:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 138:

Arg Leu Tyr Leu Gln Met Asn
1               5

(2) INFORMATION FOR SEQ ID NO: 139:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 139:

Ile Leu Tyr Leu Gln Met Asn
1               5

(2) INFORMATION FOR SEQ ID NO: 140:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 140:

Thr Leu Phe Leu Glu Met Thr
1               5

(2) INFORMATION FOR SEQ ID NO: 141:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 141:

Thr Leu Phe Leu Gln Met Thr
1               5

(2) INFORMATION FOR SEQ ID NO: 142:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 142:

Gln Val Phe Leu Lys Met Asn
1               5

(2) INFORMATION FOR SEQ ID NO: 143:

(i) SEQUENCE CHARACTERISTICS:
(A) LENGTH: 6 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 143:

Thr Ala Tyr Leu Gln Leu
1               5

(2) INFORMATION FOR SEQ ID NO: 144:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 144:

Thr Ala Tyr Met Gln Leu Ser
1               5

(2) INFORMATION FOR SEQ ID NO: 145:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 145:

Gln Val Phe Leu Lys Met Asn
1               5

(2) INFORMATION FOR SEQ ID NO: 146:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 146:

Thr Ala Thr Met Gln Leu Ser
1               5

(2) INFORMATION FOR SEQ ID NO: 147:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 147:

Gln Ile Phe Leu Lys Met Asn
1               5

(2) INFORMATION FOR SEQ ID NO: 148:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 148:

Gly Leu Tyr Phe Cys Lys Val Glu
1               5

(2) INFORMATION FOR SEQ ID NO: 149:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 149:

Gly Leu Tyr Leu Cys Lys Val Glu
1               5

(2) INFORMATION FOR SEQ ID NO: 150:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 150:

Gly Leu Tyr Val Cys Lys Val Glu
1               5

(2) INFORMATION FOR SEQ ID NO: 151:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 151:

Asp Ile Tyr Phe Cys Lys Ile Glu
1               5

(2) INFORMATION FOR SEQ ID NO: 152:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 152:

Asp Ile Tyr Phe Cys Leu Lys Glu
1               5

(2) INFORMATION FOR SEQ ID NO: 153:

(i) SEQUENCE CHARACTERISTICS:

(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 153:

Ala Thr Tyr Tyr Cys Gln Gln Trp
1               5

(2) INFORMATION FOR SEQ ID NO: 154:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 154:

Ala Val Tyr Tyr Cys Gln Asn Asn
1               5

(2) INFORMATION FOR SEQ ID NO: 155:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 155:

Ala Leu Tyr Tyr Cys Gln Gln His
1               5

(2) INFORMATION FOR SEQ ID NO: 156:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 156:

Ala Val Tyr Val Cys Gln Asn Asp
1               5

(2) INFORMATION FOR SEQ ID NO: 157:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 157:

Gly Ile Tyr Tyr Cys Val Leu Arg
1               5

(2) INFORMATION FOR SEQ ID NO: 158:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 158:

Ala Ile Tyr Tyr Cys Ala Arg Asn
1               5

(2) INFORMATION FOR SEQ ID NO: 159:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 159:

Gly Leu Tyr Tyr Cys Ala Arg Arg
1               5

(2) INFORMATION FOR SEQ ID NO: 160:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 160:

Gly Met Tyr Tyr Cys Ala Arg Trp
1               5

(2) INFORMATION FOR SEQ ID NO: 161:

(i) SEQUENCE CHARACTERISTICS:
(A) LENGTH: 8 amino acids
(B) TYPE: amino acid

(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 161:

Ala Arg Tyr Tyr Cys Ala Arg Glu
1               5

(2) INFORMATION FOR SEQ ID NO: 162:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 162:

Ala Val Tyr Tyr Cys Ala Thr Lys
1               5

(2) INFORMATION FOR SEQ ID NO: 163:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 163:

Ala Val Tyr Phe Cys Ala Arg Gly

1                   5

(2) INFORMATION FOR SEQ ID NO: 164:

     (i) SEQUENCE CHARACTERISTICS:
          (A) LENGTH: 8 amino acids
          (B) TYPE: amino acid
          (C) STRANDEDNESS: single
          (D) TOPOLOGY: linear

    (ii) MOLECULE TYPE: peptide

   (iii) HYPOTHETICAL: NO

    (iv) ANTI-SENSE: NO

    (xi) SEQUENCE DESCRIPTION: SEQ ID NO: 164:

     Ala Val Tyr Tyr Cys Ala Arg His
     1               5

(2) INFORMATION FOR SEQ ID NO: 165:

     (i) SEQUENCE CHARACTERISTICS:
          (A) LENGTH: 8 amino acids
          (B) TYPE: amino acid
          (C) STRANDEDNESS: single
          (D) TOPOLOGY: linear

    (ii) MOLECULE TYPE: peptide

   (iii) HYPOTHETICAL: NO

    (iv) ANTI-SENSE: NO

    (xi) SEQUENCE DESCRIPTION: SEQ ID NO: 165:

     Ala Xaa Tyr Tyr Cys Val Ser Tyr
     1               5

(2) INFORMATION FOR SEQ ID NO: 166:

     (i) SEQUENCE CHARACTERISTICS:
          (A) LENGTH: 8 amino acids
          (B) TYPE: amino acid
          (C) STRANDEDNESS: single
          (D) TOPOLOGY: linear

    (ii) MOLECULE TYPE: peptide

   (iii) HYPOTHETICAL: NO

    (iv) ANTI-SENSE: NO

(xi) SEQUENCE DESCRIPTION: SEQ ID NO: 174:

Tyr Trp Gly Gln Gly Thr Ser Val Thr
1               5

(2) INFORMATION FOR SEQ ID NO: 175:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 175:

Val Trp Gly Ala Gly Thr Thr Val Thr
1               5

(2) INFORMATION FOR SEQ ID NO: 176:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 176:

Tyr Trp Gly Arg Gly Thr Leu Val Thr
1               5

(2) INFORMATION FOR SEQ ID NO: 177:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 177:

Tyr Trp Gly Arg Gly Thr Leu Val Thr
1               5

(2) INFORMATION FOR SEQ ID NO: 178:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 178:

Tyr Trp Gly Gln Gly Thr Thr Leu Thr
1               5

(2) INFORMATION FOR SEQ ID NO: 179:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 179:

Tyr Trp Gly Gln Gly Thr Thr Leu Thr
1               5

(2) INFORMATION FOR SEQ ID NO: 180:

(i) SEQUENCE CHARACTERISTICS:

(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 180:

Tyr Trp Gly Gln Gly Thr Leu Val Thr
1               5

(2) INFORMATION FOR SEQ ID NO: 181:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 181:

Ser Ser His Gly Val
1               5

(2) INFORMATION FOR SEQ ID NO: 182:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 182:

Ser Ser Arg Gly Ile
1               5

(2) INFORMATION FOR SEQ ID NO: 183:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 183:

Ser Ser Arg Gly Val
1               5

(2) INFORMATION FOR SEQ ID NO: 184:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 184:

Ser Ser Arg Gly Val
1               5

(2) INFORMATION FOR SEQ ID NO: 185:

(i) SEQUENCE CHARACTERISTICS:
(A) LENGTH: 3 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 185:

Ser Asn Glu
1

(2) INFORMATION FOR SEQ ID NO: 186:

(i) SEQUENCE CHARACTERISTICS:
(A) LENGTH: 3 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 186:

Asn Asn Glu
1

(2) INFORMATION FOR SEQ ID NO: 187:

(i) SEQUENCE CHARACTERISTICS:
(A) LENGTH: 3 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 187:

Asp Asn Glu
1

(2) INFORMATION FOR SEQ ID NO: 188:

(i) SEQUENCE CHARACTERISTICS:
(A) LENGTH: 3 amino acids
(B) TYPE: amino acid

(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 188:

Asp Asn Ala
1

(2) INFORMATION FOR SEQ ID NO: 189:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 189:

Ser Pro Gly Glu Lys
1               5

(2) INFORMATION FOR SEQ ID NO: 190:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 190:

Gln Pro Gly Gly Ser

1                5

(2) INFORMATION FOR SEQ ID NO: 191:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 191:

Ser Pro Ser His Asn Thr Asp
1               5

(2) INFORMATION FOR SEQ ID NO: 192:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 192:

Ala Ser Ser His Asn Thr Asp
1               5

(2) INFORMATION FOR SEQ ID NO: 193:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 193:

Ala Ser Pro Gly Lys Ala Thr
1               5

(2) INFORMATION FOR SEQ ID NO: 194:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 194:

Glu Ser Ser Gly Lys Ala Asp
1               5

(2) INFORMATION FOR SEQ ID NO: 195:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 195:

Ala Ser His Gly Lys Ala Thr
1               5

(2) INFORMATION FOR SEQ ID NO: 196:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 196:

Ser Tyr Asn Leu Leu Ala Lys
1               5

(2) INFORMATION FOR SEQ ID NO: 197:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 197:

Thr Tyr Asn Leu Phe Ser Lys
1               5

(2) INFORMATION FOR SEQ ID NO: 198:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 198:

Ser Tyr Asn Leu Phe Ser Arg
1               5

(2) INFORMATION FOR SEQ ID NO: 199:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 199:

Thr Tyr Asn Gly Thr Gly Lys
1               5

(2) INFORMATION FOR SEQ ID NO: 200:

(i) SEQUENCE CHARACTERISTICS:
(A) LENGTH: 2 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 200:

Asn Asp
1

(2) INFORMATION FOR SEQ ID NO: 201:

(i) SEQUENCE CHARACTERISTICS:
(A) LENGTH: 2 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 201:

Asp Ser
1

(2) INFORMATION FOR SEQ ID NO: 202:

(i) SEQUENCE CHARACTERISTICS:
(A) LENGTH: 2 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 202:

Gly Ser
1

(2) INFORMATION FOR SEQ ID NO: 203:

(i) SEQUENCE CHARACTERISTICS:
(A) LENGTH: 2 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 203:

Asn Ser
1

(2) INFORMATION FOR SEQ ID NO: 204:

(i) SEQUENCE CHARACTERISTICS:
(A) LENGTH: 3 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 204:

Pro Gly Ser
1

(2) INFORMATION FOR SEQ ID NO: 205:

(i) SEQUENCE CHARACTERISTICS:
(A) LENGTH: 3 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 205:

Pro Gly Gln
1

(2) INFORMATION FOR SEQ ID NO: 206:

(i) SEQUENCE CHARACTERISTICS:
(A) LENGTH: 3 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 206:

Pro Glu Lys
1

(2) INFORMATION FOR SEQ ID NO: 207:

(i) SEQUENCE CHARACTERISTICS:

(A) LENGTH: 3 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 207:

Pro Gly Lys
1

(2) INFORMATION FOR SEQ ID NO: 208:

(i) SEQUENCE CHARACTERISTICS:
(A) LENGTH: 3 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 208:

Pro Asp Lys
1

(2) INFORMATION FOR SEQ ID NO: 209:

(i) SEQUENCE CHARACTERISTICS:
(A) LENGTH: 3 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 209:

Pro Glu Lys
1

(2) INFORMATION FOR SEQ ID NO: 210:

(i) SEQUENCE CHARACTERISTICS:
(A) LENGTH: 2 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 210:

Glu Ser
1

(2) INFORMATION FOR SEQ ID NO: 211:

(i) SEQUENCE CHARACTERISTICS:
(A) LENGTH: 2 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 211:

Gly Asn
1

(2) INFORMATION FOR SEQ ID NO: 212:

(i) SEQUENCE CHARACTERISTICS:
(A) LENGTH: 2 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 212:

Asn Glu
1

(2) INFORMATION FOR SEQ ID NO: 213:

(i) SEQUENCE CHARACTERISTICS:
(A) LENGTH: 2 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 213:

Lys Asp
1

(2) INFORMATION FOR SEQ ID NO: 214:

(i) SEQUENCE CHARACTERISTICS:
(A) LENGTH: 2 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 214:

Gly Thr
1

(2) INFORMATION FOR SEQ ID NO: 215:

(i) SEQUENCE CHARACTERISTICS:
(A) LENGTH: 4 amino acids
(B) TYPE: amino acid

(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 215:

Asp Ser Lys Ser
1

(2) INFORMATION FOR SEQ ID NO: 216:

(i) SEQUENCE CHARACTERISTICS:
(A) LENGTH: 4 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 216:

Thr Ser Gln Ser
1

(2) INFORMATION FOR SEQ ID NO: 217:

(i) SEQUENCE CHARACTERISTICS:
(A) LENGTH: 4 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 217:

Asn Ala Lys Asn

1

(2) INFORMATION FOR SEQ ID NO: 218:

(i) SEQUENCE CHARACTERISTICS:
(A) LENGTH: 4 amino acids
(B) TYPE: amino acid
(C) STRANDEDNESS: single
(D) TOPOLOGY: linear

(ii) MOLECULE TYPE: peptide

(iii) HYPOTHETICAL: NO

(xi) SEQUENCE DESCRIPTION: SEQ ID NO: 218:

Asn Pro Lys Asn
1

(2) INFORMATION FOR SEQ ID NO: 219:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 219:

Gly Leu Arg Ala Val Asp Thr
1               5

(2) INFORMATION FOR SEQ ID NO: 220:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 220:

Gly Leu Arg Ala Ala Asp Thr
1               5

(2) INFORMATION FOR SEQ ID NO: 221:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 221:

Gly Leu Arg Ala Met Asp Thr
1               5

(2) INFORMATION FOR SEQ ID NO: 222:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 222:

Gly Leu Ser Ala Met Asp Thr
1               5

(2) INFORMATION FOR SEQ ID NO: 223:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 223:

Asn Leu His Val Asn His Thr
1               5

(2) INFORMATION FOR SEQ ID NO: 224:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 224:

Asn Leu Asp Val Asn His Thr
1               5

(2) INFORMATION FOR SEQ ID NO: 225:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 225:

Asn Leu Tyr Val Asn Gln Thr
1               5

(2) INFORMATION FOR SEQ ID NO: 226:

(i) SEQUENCE CHARACTERISTICS:

(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 226:

Asp Leu Tyr Val Asn Gln Thr
1               5

(2) INFORMATION FOR SEQ ID NO: 227:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 227:

Asn Leu Phe Val Asn Gln Thr
1               5

(2) INFORMATION FOR SEQ ID NO: 228:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 228:

Asn Met Ser Ala Ser Gln Thr
1               5

(2) INFORMATION FOR SEQ ID NO: 229:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 229:

Leu Met Tyr Pro Pro Pro Tyr Phe Val
1               5

(2) INFORMATION FOR SEQ ID NO: 230:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 230:

Leu Met Tyr Pro Pro Pro Tyr Tyr Leu
1               5

(2) INFORMATION FOR SEQ ID NO: 231:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 231:

Leu Met Tyr Pro Pro Pro Tyr Tyr Val
1               5

(2) INFORMATION FOR SEQ ID NO: 232:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 232:

Phe Met Tyr Pro Pro Pro Tyr Leu Asp Asn
1               5                   10

(2) INFORMATION FOR SEQ ID NO: 233:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 233:

Val Met Tyr Pro Pro Pro Tyr Leu Asp Asn
1               5                   10

(2) INFORMATION FOR SEQ ID NO: 234:

(i) SEQUENCE CHARACTERISTICS:
(A) LENGTH: 10 amino acids
(B) TYPE: amino acid

(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 234:

Val Leu Tyr Pro Pro Pro Tyr Ile Asp Asn
1               5                   10

(2) INFORMATION FOR SEQ ID NO: 235:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 235:

Val Met Tyr Pro Pro Pro Tyr Ile Gly Asn
1               5                   10

(2) INFORMATION FOR SEQ ID NO: 236:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(xi) SEQUENCE DESCRIPTION: SEQ ID NO: 236:

Ala Met Tyr Pro Pro Pro Tyr Val Tyr Asn

1                    5                    10